(19) **Europäisches Patentamt / European Patent Office / Office européen des brevets**

(11) **EP 4 287 198 A1**

(12) # EUROPEAN PATENT APPLICATION

(43) Date of publication:
**06.12.2023 Bulletin 2023/49**

(21) Application number: **22177285.8**

(22) Date of filing: **03.06.2022**

(51) International Patent Classification (IPC):
**G16H 20/70** (2018.01)  **G16H 50/30** (2018.01)
**A61B 5/16** (2006.01)

(52) Cooperative Patent Classification (CPC):
**G16H 20/70; G16H 50/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Neural Assembly Int AB**
**100 61 Stockholm (SE)**

(72) Inventors:
• **KLINGBERG, Torkel**
  **113 38 Stockholm (SE)**
• **ERICSON, Julia**
  **114 23 Stockholm (SE)**

(74) Representative: **Valea AB**
**Box 1098**
**405 23 Göteborg (SE)**

(54) **METHOD AND SYSTEM FOR DETERMINING WHICH STAGE A USER PERFORMANCE BELONGS TO**

(57) The present disclosure relates to a computer training system (100) for determining which stage a user performance belongs to. The system (100) is arranged to present multiple trials. The system (100) is arranged to receive user input to each trial at a respective time instant. The user input is associated with a first type and a second type of user improvement. The system (100) is arranged to determine a user performance at each time instant. The user performance comprises a first stage or a second stage of user performance. The first stage comprises the first type and the second type of user improvement. The second stage comprises the second type of user improvement, but not the first type. The system (100) is arranged to determine an indication of whether the user performance at each time instant belongs to the first stage or the second stage of user performance.

100

108. Input unit

110. Output unit

105. Processor

103. Memory

101. Computer

**Fig. 1**

EP 4 287 198 A1

**Description**

TECHNICAL FIELD

The present disclosure relates generally to a computer training system and a method performed by the computer training system. More particularly, the present disclosure relates to determining which stage a user performance belongs to.

BACKGROUND

A focus of cognitive training research has been to understand if training can lead to an enhancement of general cognitive abilities, or if improvements are merely generated by task specific strategy development. An important but often overlooked aspect of this research is the temporal dynamics of the performance gains during training. Such dynamics could help to separate different types of cognitive improvements, providing a more nuanced description of the process which extends beyond the binary question "did the training lead to transfer?". Therefore, it is of interest to identify and separate cognitive training processes. A fast, task specific improvement usually takes place within the first few days of training while the effect on general cognitive capacities evolves slowly but constantly throughout the training period. This highlights the fact that important improvements could still occur late in the training process even though the typically logarithmic learning curves would suggest that the first days of training are the most eventful. Today, identification and separating of the cognitive training process is done by psychological tests conducted by for example psychologists or other suitable testing personnel. These psychological tests are demanding and tiering for the test person and requires a lot of resources with respect for example the testing personnel. Psychological tests also expensive to conduct.

For more than a century, a central question in cognitive psychology has been when, how and to what extent training transfers to non-trained tasks or behaviors. But there is still a lack of mathematical models to describe the processes underlying cognitive training and transfer.

In an attempt to bring clarity to the debate, transfer has often been categorized as either "near" or "far". Near transfer often refers to improvement on the trained task or tasks very similar to it. Such improvement could be the result of implicit leaming, such as perceptual learning. Near transfer could also be the result of explicit strategies, such as associating numbers in working memory (WM) to long-term memories, which leads to large and quick improvement on the trained task but do not transfer, for example to improvement on WM for letters. Far transfer, on the other hand, refers to an improvement on a more general capacity, such as WM capacity, verbal or spatial ability, general fluid intelligence (gF) or g, which would be useful for a wider range of non-trained tasks.

A weakness of the near/far categorization is that it is at best imprecise and often arbitrary. Secondly, the near/far terminology infers a dichotomy, which is at odds with models of mental abilities that suggest multiple levels of gradual generality from task specific skills to general intelligence (g) or multiple dimensions. This false dichotomy is also reflected in many meta-analyses, where widely heterogeneous tasks are grouped into a "far" transfer category. This can result in a lack of significant effect, leading to the false conclusion that there was only near transfer. Factor analysis of transfer task could possibly be a better way to characterize and quantify transfer, and it has previously been used in cognitive training studies training studies.

Another shortcoming of transfer measures is that they often fail to describe the temporal dynamics of training. Such temporal dynamics should be a fundamental part of cognitive training studies as they provide a way to identify and separate processes occurring during training. An example of this is found in motor training, where repeated functional magnetic resonance imaging (fMRI) during the training process revealed two different types of neural changes occurring on different time scales. A fast improvement was related to the development of task specific routines, while a slower reorganization of the motor cortex was related actual improvements of motor skills.

Studies of temporal dynamics have also suggested that there are different phases in mathematical skill acquisition. In these studies, a piecewise continuous power function has been fitted to performance data in mathematical learning using a hidden Markov model (HMM). The HMM evaluates, in each trial, the probability of transitioning from one learning phase to the next, where each phase has a distinct intercept and learning rate. Using this method, three phases of mathematical skill acquisition were found. Importantly, the three phases were only identified when the HMM was fitted to individual training data, while only one phase was found on a group level. This highlights the importance of analyzing temporal dynamics using individual learning curves, as averaging can give the false impression of smoothness. Phases of training, reflecting different neural processes, has thus been identified through analysis of time-dynamics in both motor and mathematical training.

Therefore, there is a need to at least mitigate, improve or solve this issue.

SUMMARY

An object is to obviate at least one of the above disadvantages and to improve the detection of which stage a user performance belongs to.

According to a first aspect, the object is achieved by a computer training system of claim 1. The computer training system is for determining which stage a user performance belongs to. The computer training system is arranged to present multiple trials on a display. The multiple trials are presented in a time consecutive order and comprising varying difficulty levels. The difficulty level for each trial is defined by an amount of data to be retained in a working memory of a user when handling the trial and adapted to gradual improvement in user performance. The computer training system is arranged to receive user input to each trial at a respective time instant. The user input is associated with a first type of user improvement and a second type of user improvement. The computer training system is arranged to determine, based on the user input a user performance at each time instant. The user performance comprises a first stage of user performance or a second stage of user performance. The first stage comprises the first type of user improvement and the second type of user improvement. The second stage comprises the second type of user improvement but not the first type of user improvement. The computer training system is arranged to determine an indication of whether the user performance at each time instant belongs to the first stage of user performance or the second stage of user performance.

According to a second aspect, the object is achieved by a method of claim 10. The method is performed by a computer training system for determining which stage a user performance belongs to. The computer training system presents multiple trials on a display. The multiple trials are presented in a time consecutive order and comprising varying difficulty levels. The difficulty level for each trial is defined by an amount of data to be retained in a working memory of a user when handling the trial and adapted to gradual improvement in user performance. The computer training system receives user input to each trial at a respective time instant. The user input is associated with a first type of user improvement and a second type of user improvement. The computer training system determines, based on the user input a user performance at each time instant. The user performance comprises a first stage of user performance or a second stage of user performance. The first stage comprises the first type of user improvement and the second type of user improvement, and the second stage comprises the second type of user improvement but not the first type of user improvement. The computer training system determines an indication of whether the user performance at each time instant belongs to the first stage of user performance or the second stage of user performance.

According to a third aspect, the object is achieved by a computer program of claim 13. The computer program comprises program code means for performing the method of the second aspect when the computer program is run on a computer

According to a fourth aspect, the object is achieved by a computer readable medium of claim 14. The computer readable medium carries a computer program comprising program code means for performing the method of the second aspect when the computer program is run on a computer.

The present disclosure herein affords many advantages, of which a non-exhaustive list of examples follows: An advantage of the present disclosure may be that it makes it possible to determine whether a user performance belongs to a first stage or second stage in a fast and reliable way.

Another advantage of present disclosure may be that it may give a measure of the amount of improvement in the second stage, without administering psychological tests before, during and after training.

Individuals differ in how much they improve. Some individuals thus need more training than others to achieve the same amount of improvement. Another advantage may be that the analysis of improvement in the second stage may be used to suggest if further training is necessary for a particular individual.

The present disclosure is not limited to the features and advantages mentioned above. A person skilled in the art will recognize additional features and advantages upon reading the following detailed description.

BRIEF DESCRIPTION OF THE DRAWINGS

The present disclosure will now be described in more detail by way of example only in the following detailed description by reference to the appended drawings in which:

Fig. 1    is a schematic drawing illustrating a computer training system.
Fig. 2    is a flow chart illustrating a method.
Fig. 3    are graphs illustrating a piecewise linear model fitted to different users.

The drawings are not necessarily to scale, and the dimensions of certain features may have been exaggerated for the sake of clarity. Emphasis is instead placed upon illustrating the principle.

DETAILED DESCRIPTION

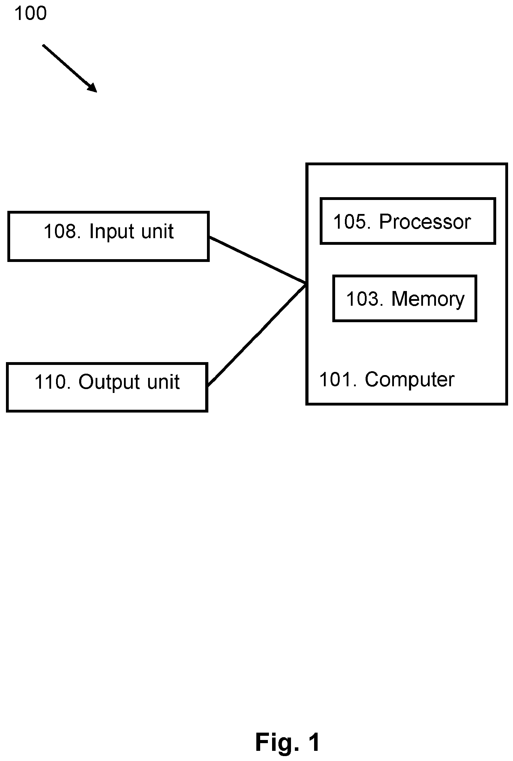

Fig. 1 is a schematic block diagram illustrating a computer training system 100. The computer training system 100 comprises a **computer 101.** The computer 101 may be a personal computer (PC), a workstation computer, a mobile phone, a tablet computer or some other type of computer, to be used by a user for training and improving his/her working memory capacity. The user may have various reasons for the need to train and improve his/her working memory capacity such as for example, that he/she has a working memory deficit, e.g. ADHD, a brain damage etc. The user may be an elderly person having an impaired capacity due to age or it may be any other person wanting to train and improve his/her working memory capacity. At the same time as the user trains and improves his /hers working memory capacity, the computer training system 100 is arranged to determine which stage a user performance belongs to.

The computer 101 is arranged to comprise a computer program for determining which stage a user performance belongs to. The computer 101 is arranged to comprise a computer program for training the working memory of the user, e.g. by presenting trials that a user should handle or answer. The computer program(s) is/are arranged to be executed on the computer 101.

The computer program(s) is/are stored in a **memory 103** associated with the computer 101. The memory 103 may comprise one or more memory units. The memory 103 is arranged to be used to store obtained information, store data, configurations, schedulings, and applications etc. to perform the methods herein when being executed in the computer 101. The memory 103 may be completely comprised in the computer 101, it may be partly comprised in the computer 101 and partly comprised in another location, e.g. a cloud memory, or it may be completely comprised in a cloud memory.

The computer 101 comprises one or more processors 110, or the computer 101 may be a processor. The processor may be for example a Digital Signal Processor (DSP), Application Specific Integrated Circuit (ASIC) processor, Field-programmable gate array (FPGA) processor or microprocessor. The program code mentioned above may also be provided as a computer program product, for instance in the form of a data carrier carrying computer program code for performing the present disclosure herein when being loaded into the computer 101. One such carrier may be in the form of a CD ROM disc. It is however feasible with other data carriers such as a memory stick. The computer program code can be provided as pure program code on a server and downloaded to the computer 101.

The computer 101 is arranged to be connected to one or more **input units 108.** The one or more input units 108 is arranged to receive input into the computer, for example input in response to trials performed by a user. The one or more input units 108 may be a keyboard, a touch display, a pointing device, a microphone etc.

The computer 101 is arranged to be connected to one or more **output units 110.** The one or more output units 110 is arranged to output, e.g. present or display, trials, information etc. to the user. The one or more output units 108 is arranged to present answers to the trials when handled by the user. The one or more output units 110 may be a display, a touch display, a speaker etc.

The input units 108 and the output units 110 may be referred to as communication interface units.

It should be noted that the communication links in the computer training system 100 may be of any suitable kind comprising either a wired or wireless link. The link may use any suitable protocol depending on type and level of layer, e.g. as indicated by the Open Systems Interconnection (OSI) model, as understood by the person skilled in the art.

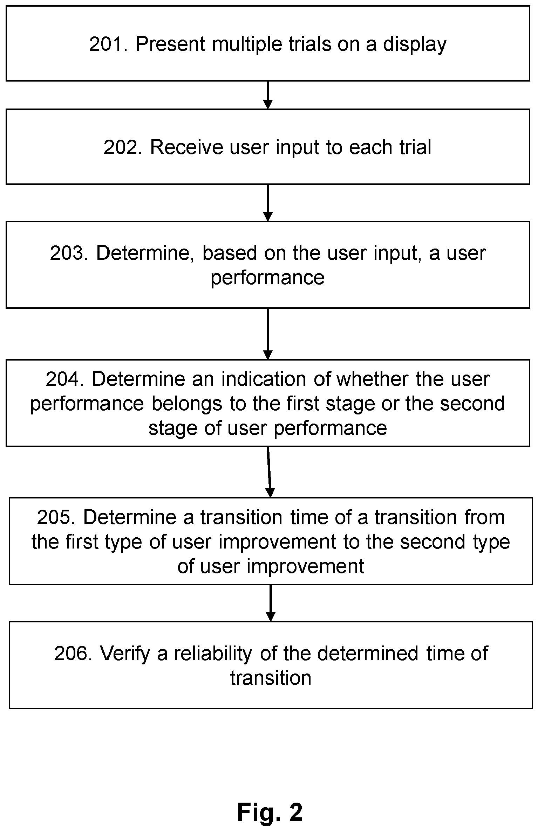

The method for determining which stage a user performance belongs to, will now be described with reference to the flowchart depicted in fig. 2. The method comprises the following steps, which steps may as well be carried out in another suitable order than described below.

Step 201

The computer training system 100 presents multiple trials on a display. The multiple trials are presented in a time consecutive order and comprising varying difficulty levels. The difficulty level for each trial is defined by an amount of data to be retained in a working memory of a user when handling the trial and adapted to gradual improvement in user performance. A trial may be referred to as a task, a job, information to be handled by the user. Instead of or in addition to presenting the trials on the display, the computer training system 100 may present the multiple trials via voice outputted from a speaker or by means of a tactile output.

An amount of data to be retained in the working memory of the user may correspond to a difficulty level of the trial.

The trial is arranged to be handled by a user by any suitable means, for example by typing an answer using a keyboard, by answering using voice, by touching a touch display etc.

The trials may have increased difficulty level, or a mix of one or more of: decreasing, constant and increased difficulty level.

One trial may be presented to the user every day in a period of for example 10 days, or 10 trials may be presented to the user three days a week for a period of 5 weeks etc.

A purpose of the trials may be to improve the working memory of the user.

Step 202

The computer training system 100 receives user input to each trial at a respective time instant t. The user input is associated with a first type of user improvement and a second type of user improvement. The user input may be an answer to a trial.

The first type of user improvement may be related to user strategy in handling the trial, e.g. a specific trial, and the second type of user improvement may be related to user capacity in handling the trial, e.g. a more general user cognitive capacity in handling the trial.

The computer training system 100 may create training data comprising the user input for each trial at each time and the corresponding difficulty level for each trial.

There may be a change in user input to each trial, e.g. a change in its performance.

The computer training system 100 may determine if the user input is a correct or incorrect answer to the trial.

Step 203

The computer training system 100 determines, based on the user input a user performance at each time instant. The user performance comprises a first stage of user performance or a second stage of user performance. The first stage comprises the first type of user improvement and the second type of user improvement. The second stage comprises the second type of user improvement but not the first type of user improvement.

The first stage of user performance may be represented by

$$y = \beta_{cog}t + \beta_{strat}t + \alpha + w$$

The second stage of user performance may be represented by

$$y = \beta_{cog}t + \Delta_{strat} + \alpha + w,$$

where

- y is the user performance, $\beta_{cog}$ is a coefficient for the second type of user improvement,
- $\beta_{strat}$ is a coefficient for the first type of user improvement,
- $\Delta_{strat}$ is a total first type of user improvement up until the time of transition from the first stage to the second stage,
- $t$ is the time of receipt of the user input,
- $\alpha$ is an initial user performance, and
- $w$ is white noise with standard deviation $\sigma$.

Step 204

The computer training system 100 determines an indication of whether the user performance at each time instant belongs to the first stage of user performance or the second stage of user performance.

The computer training system 100 may determine the indication of whether the user performance at each time instant belongs to the first stage of user performance or the second stage of user performance by applying a Hidden Markov Model, HMM.

The indication of whether the user performance at each time instant belongs to the first stage of user performance or the second stage of user performance may be determined by the computer training system 100 by:

- determining a probability of a certain type of user performance at the time instant belonging to the first stage of user performance by using

$$p(y_t|x_t) = \mathcal{N}(\beta_{cog}t + \beta_{strat}t + \alpha, \sigma)$$ ;

- determining a probability of a certain type of user performance at the time instant belonging to the second stage of user performance using:

$$p(y_t|x_t) = \mathcal{N}(\beta_{cog}t + \Delta_{strat} + \alpha, \sigma)$$

and

- determining a probability that a transition from the first stage of user performance to the second stage of user performance having occurred at a certain time instant using:

$$p(\text{trans} = t) = (1 - \tau)^t \cdot p(y_1, \ldots, y_t|x = 1) \cdot \tau \cdot p(y_{t+1}, \ldots, y_N|x = 2)$$

A probability of a transition from the first stage of user performance to the second stage of user performance may be $p(x_t|x_{t-1}) = \tau$,
where

- $x_t$ is the certain stage at the time instant,
- the certain stage is the first stage or the second stage, and
- $y_t$ is the user performance at the time instant.

$y_t$ may be described as the user performance, e.g. a correct or incorrect answer to the trial, or an average level during a time period, e.g. a day, or an average correctness during a day.

Step 205

The computer training system 100 may determine a transition time of a transition from the first type of user improvement to the second type of user improvement for the user performance at each time instant.

Step 206

The computer training system 100 may verify a reliability of the determined time of transition.
The reliability may be verified by determining if the determined time of transition comprises a bias and by assessing a size of prediction errors.

Step 207

The computer training system 100 may, when the user performance belongs to the second stage and is above a threshold, determine that one or more further trials should be presented on the display. The threshold may be predetermined or dynamically determined during execution of the method. The step may comprise to determine how many further trials that should be presented on the display.
Users differ in how much they improve. Some users thus need more training than others to achieve the same amount of user improvement. The computer training system 100 may therefore suggest if further training is necessary for a particular user.

Step 208

The computer training system 100 may present one or more further trials on the display.

Step 209

The computer training system 100 may select which one or more further trials from a plurality of available further trials that should be presented on the display. The available further trials have varying difficulty level. The selected one or more further trials may be of a difficulty level that corresponds a level of the user performance belonging to the second stage. For example if the user performance belonging to the second stage is of a level 1, then the selected one or more further trials may be of a difficulty level 1. If the user performance belonging to the second stage is of a level 2, then the selected one or more further trials may be of a difficulty level 2. Thus, the higher the user performance is, the higher the difficulty level may be.
The computer training system 100 for determining which stage a user performance belongs to is arranged to perform the method in fig. 2.

Some further details of the above will now be provided.

**Fitting individual learning curves to daily training data**

When the computer training system 100 performs a confirmatory factor analysis (CFA), based on the sparsely collected transfer measures, it may determine that training improvement is comprised of two different factors - task specific strategy improvements and a common cognitive capacity improvement. Cognitive capacity improvements were present throughout the training period while the strategy improvements happened between the first and the second transfer test occasions. Next, the computer training system 100 may assume that these processes may be reflected in the learning curve of the trial and evaluated using a mathematical model, fitted to the daily performance data, which may: 1) reflect this dual-process improvement, 2) correlate with the CFA measures, and 3) provide more detailed information about the temporal dynamics of these processes.

**A Piecewise Linear Model**

With the CFA, the computer training system 100 may not only suggest a two-factor model, but also two different stages, where the first stage comprised both a strategy improvement and an improvement of the cognitive factor while the second stage only included a linear cognitive increase. The computer training system 100 may use a piecewise linear (PL) function to describe this. Therefore, a simple way for the computer training system 100 to model daily training performance may be through a PL model with the following two stages:

$$\text{Stage 1:} \quad y = \beta_{cog} t + \beta_{strat} t + \alpha$$

$$\text{Stage 2:} \quad y = \beta_{cog} t + \Delta_{strat} + \alpha$$

$\beta_{cog}$ and $\beta_{strat}$ are the coefficients for cognitive and strategy improvements. $\Delta_{strat}$ is the total strategy improvement up until the timepoint when the model switches from stage 1 to stage 2 times, and $y$ is the performance.

To fit the PL model, the computer training system 100 may use an HMM. An HMM is a probabilistic graphical model of a system, where the true state of the system is hidden but a variable related to the hidden state can be measured. If the relationship between the true states and the measured variables as well as the likelihood of moving from one state to another are known, the most probable state at each timepoint may be calculated using a series of observable measurements. In this case, the observable measurements may be the performances $y$ on each day. Whether a training day belongs to the first or second stage, may be the hidden information that cannot be directly observed. By optimizing the parameters of the model, the most likely day for transitioning from stage 1 to stage 2 may be determined. Optimization of the HMMs may be done by the computer training system 100 using an algorithm called expectation maximization.

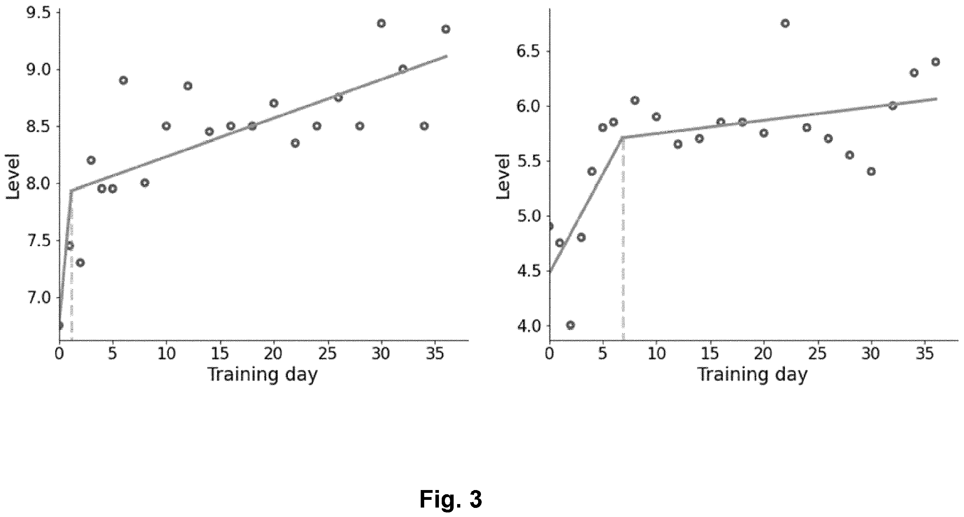

One learning curve may be fitted for each of the user that the trials are presented to. The estimates may differ from user to user and **fig. 3** shows examples of fitted learning curves for two users. The dotted lines indicate a time of transition from stage 1 to stage 2. The x-axis represents training day, and the y-axis represents level. Next, the parameters $\beta_{cog}$ and $\Delta_{strat}$ may be correlated with the two parameters extracted from the latent factor model. $\Delta_{strat}$ correlated significantly with the strategy increase in the latent factor model (r=0.73, p < $10^{-5}$), but also slightly with the cognitive capacity increase (r=0.22, p < $10^{-5}$). $\beta_{cog}$ only correlated with the cognitive capacity increase (r=0.53, p < $10^{-5}$) and not with the strategy improvement (r=0.02, p= 0.47). Thus, the estimates based on daily training data were consistent with the CFA estimates. Moreover, with the HMM, the computer training system 100 may suggest that a ceiling for the strategy was on average reached after 2.92 days of training comprising one or more trials.

To test the reliability of the determined time of transition, the computer training system 100 may fit them to simulated data where the true parameters for each subject were known. The computer training system 100 may determine if there was bias is the estimations and assess the size of the prediction errors. The computer training system 100 may add gaussian noise to the performance $y$, where the noise level on each day may be the average noise level for the real data on that day.

For the piecewise linear model, the bias for the three parameters $\beta_{cap}$, $\beta_{strat}$ and $\alpha$ may be -0.00039, 0.092 and -0.14. The respective root mean square errors (RMSE) may be 0.0069, 0.22 and 0.39. The bias in transition day may be -0.37 days with an RMSE of 0.58, see fig. 3. In 88 percent of the cases, the computer training system 100 may predict a transition day which was within a one-day span of the true transition day.

**Piecewise linear model**

The computer training system 100 may fit the PL learning curve using an HMM. The computer training system 100 may assume that the learning trajectory of WM Grid may be described using the following function with two consecutive stages:

$$\text{Stage 1:} \quad y = \beta_{cog}t + \beta_{strat}t + \alpha + w$$

$$\text{Stage 2:} \quad y - \beta_{cog}t + \Delta_{strat} + \alpha + w$$

where w is white noise with standard deviation $\sigma$. The probability of observing a certain performance y on day $t$ may be

$$p(y_t|x_t) = \mathcal{N}(\beta_{cog}t + \beta_{strat}t + \alpha, \sigma)$$

if $t$ is in state 1 and

$$p(y_t|x_t) = \mathcal{N}(\beta_{cog}t + \Delta_{strat} + \alpha, \sigma)$$

if $t$ is in state 2. Here, $x_t$ is the state on the given day. The probability of transitioning from state 1 to state 2 may be

$$p(x_t|x_{t-1}) = \tau .$$

Once the transition has occurred, the probability of returning to the first state may be zero. It should be noted that the probability of performance $y_t$ may only depend on the state ($x_t = 1$ or $x_t = 2$) on that specific day. Likewise, the probability of transition into state 2 on day $t$ may only depend on the state on day $t$ - 1. Given these assumptions as well as the assumption that the participant always starts in state 1, the computer training system 100 may describe the probability of transitioning on day $t$ as

$$p(\text{trans} = t) = (1 - \tau)^t \cdot p(y_1, \ldots, y_t|x = 1) \cdot \tau \cdot p(y_{t+1}, \ldots, y_N|x = 2) .$$

By knowing the probability of transitioning on every day, the computer training system 100 may calculate the probability of a day being in a certain state $p(x_t| y_1, ..., y_N)$ by summing together all transition day probabilities that allow for that day to be in the given state. These expected states may thereafter be used in an algorithm called Expectation Maximization, where the parameters may be optimized in an iterative process. In each iteration, the expected states may first be calculated. Then, the parameters may be optimized such that the probability of observing a sequence of performances **y** are maximized for the expected states. The iterations may continue until the algorithm has converged. The maximization step may be optimized using any suitable function.

For the maximization step, the computer training system 100 may assume that the strategy could not be negative as it is unusual to adopt a successively worse strategy during training. However, the capacity factor may be allowed to take on negative values since for example a lack of motivation at the user may be something that is transferable to other exercises. Finally, the transition day may be constrained by the findings from the CFA, which may suggest that the transition day occurred somewhere between day one and nine.

Summarized, the present disclosure enables to identify and separate cognitive training processes for users when being faced with a training program comprising multiple trials. A fast, task specific improvement arose within the first few days of training while the effects on general cognitive capacities evolved slowly but constantly throughout the training period. This highlights the fact that important improvements could still occur late in the training process even though the typically logarithmic learning curves would suggest that the first days of training are the most eventful.

The present disclosure:

1) characterizes processes involved in cognitive training using confirmatory factor analysis (CFA). Performance in both the trained and the transfer tasks are included to identify task specific as well as transferable improvements, and
2) isolates these processes in individual learning curves of the trained exercise in order to determine the temporal dynamics of the processes in more detail.

The present disclosure is not limited to the above. Various alternatives, modifications and equivalents may be used. Therefore, disclosure herein should not be taken as limiting the scope. A feature may be combined with one or more other features.

In general, the usage of "first", "second", "third", "fourth", and/or "fifth" herein may be understood to be an arbitrary way to denote different elements or entities and may be understood to not confer a cumulative or chronological character to the nouns they modify, unless otherwise noted, based on context.

The term "at least one of A and B" should be understood to mean "only A, only B, or both A and B.", where A and B are any parameter, number, indication used herein etc.

It should be emphasized that the term "comprises/comprising" when used in this specification is taken to specify the presence of stated features, integers, steps or components, but does not preclude the presence or addition of one or more other features, integers, steps, components or groups thereof. It should also be noted that the words "a" or "an" preceding an element do not exclude the presence of a plurality of such elements.

The term "configured to" used herein may also be referred to as "arranged to", "adapted to", "capable of" or "operative to".

## Claims

1. A computer training system (100) for determining which stage a user performance belongs to, the computer training system (100) being arranged to:
present multiple trials on a display, wherein the multiple trials are presented in a time consecutive order and comprising varying difficulty levels, wherein the difficulty level for each trial is defined by an amount of data to be retained in a working memory of a user when handling the trial and adapted to gradual improvement in user performance;

    receive user input to each trial at a respective time instant, wherein the user input is associated with a first type of user improvement and a second type of user improvement;
    determine, based on the user input, a user performance at each time instant, wherein the user performance comprises a first stage of user performance or a second stage of user performance, wherein the first stage comprises the first type of user improvement and the second type of user improvement, and wherein the second stage comprises the second type of user improvement but not the first type of user improvement; and to
    determine an indication of whether the user performance at each time instant belongs to the first stage of user performance or the second stage of user performance.

2. The computer training system (100) according to claim 1, arranged to
determine a transition time of a transition from the first type of user improvement to the second type of user improvement for the user performance at each time instant.

3. The computer training system (100) according to any of the preceding claims, arranged to determine the indication of whether the user performance at each time instant belongs to the first stage of user performance or the second stage of user performance by applying a Hidden Markov Model, HMM.

4. The computer training system (100) according to any of the preceding claims, wherein the first stage of user performance is represented by

$$y = \beta_{cog}t + \beta_{strat}t + \alpha + w,$$

and
wherein the second stage of user performance is represented by

$$y = \beta_{cog}t + \Delta_{strat} + \alpha + w,$$

wherein y is the user performance, $\beta_{cog}$ is a coefficient for the second type of user improvement, $\beta_{strat}$ is a coefficient for the first type of user improvement, $\Delta_{strat}$ is a total first type of user improvement up until the time of transition from the first stage to the second stage, $t$ is the time of receipt of the user input, $\alpha$ is an initial user performance and

*w* is white noise with standard deviation σ.

5. The computer training system (100) according to claim 4, wherein the indication of whether the user performance at each time instant belongs to the first stage of user performance or the second stage of user performance is determined by:

determining a probability of a certain type of user performance at the time instant belonging to the first stage of user performance by using

$$p(y_t | x_t) = \mathcal{N}(\beta_{cog} t + \beta_{strat} t + \alpha, \sigma)$$

;

determining a probability of a certain type of user performance at the time instant belonging to the second stage of user performance using:

$$p(y_t | x_t) = \mathcal{N}(\beta_{cog} t + \Delta_{strat} + \alpha, \sigma)$$

;

and
determining a probability that a transition from the first stage of user performance to the second stage of user performance having occurred at a certain time instant using:

$$p(\text{trans} = t) = (1 - \tau)^t \cdot p(y_1, \ldots, y_t | x = 1) \cdot \tau \cdot p(y_{t+1}, \ldots, y_N | x = 2)$$

wherein a probability of a transition from the first stage of user performance to the second stage of user performance is $p(x_t | x_{t-1}) = \tau$,
wherein $x_t$ is the certain stage at the time instant, and wherein the certain stage is the first stage or the second stage
$y_t$ is the user performance at the time instant.

6. The computer training system (100) according to any of the preceding claims, arranged to verify a reliability of the determined time of transition.

7. The computer training system (100) according to claim 6. wherein the reliability is verified by determining if the determined time of transition comprises a bias and by assessing a size of prediction errors.

8. The computer training system (100) according to any of the preceding claims, wherein the first type of user improvement is related to user strategy in handling the trial and the second type of user improvement is related to user capacity in handling the trial.

9. The computer training system (100) according to any of the preceding claims, wherein an amount of data to be retained in the working memory of the user corresponds to a difficulty level of the trial.

10. The computer training system (100) according to any of the preceding claims, being arranged to:

when the user performance belongs to the second stage and is above a threshold, determine that one or more further trials should be presented on the display; and
presenting one or more further trials on the display.

11. The computer training system (100) according to any of the preceding claims, being arranged to:
select which one or more further trials from a plurality of available further trials that should be presented on the display, wherein the available further trials have varying difficulty level, and wherein the selected one or more further trials is of a difficulty level that corresponds a level of the user performance belonging to the second stage.

12. A method performed by a computer training system (100) for determining which stage a user performance belongs to, the method comprising:

presenting (201) multiple trials on a display, wherein the multiple trials are presented in a time consecutive order and comprising varying difficulty levels, wherein the difficulty level for each trial is defined by an amount of data to be retained in a working memory of a user when handling the trial and adapted to gradual improvement in user performance;

receiving (202) user input to each trial at a respective time instant, wherein the user input is associated with a first type of user improvement and a second type of user improvement;

determining (203), based on the user input, a user performance at each time instant, wherein the user performance comprises a first stage of user performance or a second stage of user performance, wherein the first stage comprises the first type of user improvement and the second type of user improvement, and wherein the second stage comprises the second type of user improvement but not the first type of user improvement; and to

determining (204) an indication of whether the user performance at each time instant belongs to the first stage of user performance or the second stage of user performance.

13. A computer program comprising program code means for performing the steps of claim 12 when the computer program is run on a computer.

14. A computer readable medium carrying a computer program comprising program code means for performing the steps of claims 12 when the computer program is run on a computer.

100

108. Input unit

110. Output unit

101. Computer

105. Processor

103. Memory

**Fig. 1**

201. Present multiple trials on a display

202. Receive user input to each trial

203. Determine, based on the user input, a user performance

204. Determine an indication of whether the user performance belongs to the first stage or the second stage of user performance

205. Determine a transition time of a transition from the first type of user improvement to the second type of user improvement

206. Verify a reliability of the determined time of transition

**Fig. 2**

Fig. 3

Europäisches Patentamt

European Patent Office

Office européen des brevets

# EUROPEAN SEARCH REPORT

Application Number

EP 22 17 7285

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/210955 A1 (SKOGLUND DAVID [SE] ET AL) 21 September 2006 (2006-09-21) * The whole document, in particular: Paragraphs [0002], [0030], [0034], [0035], [0037], [0055], [0056], [0058], [0063], [0064], [0073], [0100]; Figures 2 – 4. * | 1-14 | INV. G16H20/70 G16H50/30 A61B5/16 |
| X | US 2015/031009 A1 (KULLOK JOSE ROBERTO [IL] ET AL) 29 January 2015 (2015-01-29) * The whole document, in particular: Paragraphs [0001], [0013] – [0022], [0032], [0203] – [0218]. * | 1-14 | |
| A | DONNER YONI ET AL: "Piecewise power laws in individual learning curves", PSYCHONOMIC BULLETIN, SPRINGER US, NEW YORK, vol. 22, no. 5, 25 February 2015 (2015-02-25), pages 1308-1319, XP035549106, ISSN: 1069-9384, DOI: 10.3758/S13423-015-0811-X [retrieved on 2015-02-25] * The whole document, in particular: Abstract; Pages 1308 – 1310; Figure 1. * | 1-14 | TECHNICAL FIELDS SEARCHED (IPC) G16H A61B |
| A | JUDD NICHOLAS ET AL: "Training spatial cognition enhances mathematical learning in a randomized study of 17,000 children", NATURE HUMAN BEHAVIOUR, NATURE PUBLISHING GROUP UK, LONDON, vol. 5, no. 11, 20 May 2021 (2021-05-20), pages 1548-1554, XP037622805, DOI: 10.1038/S41562-021-01118-4 [retrieved on 2021-05-20] * The whole document, in particular: Abstract; Figure 2. * | 1-14 | |

-/--

The present search report has been drawn up for all claims

| Place of search | Date of completion of the search | Examiner |
|---|---|---|
| Munich | 7 November 2022 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MINEAR MEREDITH ET AL: "A simultaneous examination of two forms of working memory training: Evidence for near transfer only", MEMORY, SPRINGER US, NEW YORK, vol. 44, no. 7, 29 April 2016 (2016-04-29), pages 1014-1037, XP036150203, ISSN: 0090-502X, DOI: 10.3758/S13421-016-0616-9 [retrieved on 2016-04-29] * The whole document, in particular: Abstract; Figure 1. * | 1-14 | |

-----

TECHNICAL FIELDS
SEARCHED     (IPC)

The present search report has been drawn up for all claims

| Place of search | Date of completion of the search | Examiner |
|---|---|---|
| Munich | 7 November 2022 | Nagele, Stefan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

...................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 17 7285

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2022

| Patent document cited in search report | | Publication date | Patent family member(s) | | Publication date |
|---|---|---|---|---|---|
| US 2006210955 | A1 | 21-09-2006 | AU | 2007267776 A1 | 06-12-2007 |
| | | | CA | 2653216 A1 | 06-12-2007 |
| | | | EP | 2030183 A2 | 04-03-2009 |
| | | | US | 2006210955 A1 | 21-09-2006 |
| | | | WO | 2007140064 A2 | 06-12-2007 |
| US 2015031009 | A1 | 29-01-2015 | US | 2015031003 A1 | 29-01-2015 |
| | | | US | 2015031009 A1 | 29-01-2015 |
| | | | US | 2015031010 A1 | 29-01-2015 |
| | | | US | 2015086950 A1 | 26-03-2015 |

EPO FORM P0459

For more details about this annex : see Official Journal of the European Patent Office, No. 12/82